# EUROPEAN PATENT APPLICATION

(11) **EP 2 732 835 A1**
(43) Date of publication of application: **21.05.2014**
(21) Application number: 12192514.3
(22) Date of filing: 14.11.2012
(51) Int. Cl.: A61M 5/00

(54) **Needle assembly magazine**

(71) Applicant: Sanofi-Aventis Deutschland GmbH, 65929 Frankfurt am Main (DE)
(72) Inventor: The designation of the inventor has not yet been filed

(57) **Abstract**

Described is a needle assembly magazine (1) comprising a body (2), and a plurality of needle assembly compartments (3) formed in the body (2). Each needle assembly compartment (3) includes at least one track (19) adapted to receive a follower pin (25) coupled to a needle assembly (4). The at least one track (19) comprises a substantially horizontal proximal portion (20) coupled to a substantially vertical longitudinal portion (22) coupled to a substantially horizontal distal portion (21). The needle assembly (4) is restricted to rotational movement relative to the needle assembly compartment (3) when the follower pin (25) is in the proximal portion (20) and the distal portion (21) and restricted to axial movement when the follower pin (25) is in the longitudinal portion (22).

## Description

### Technical Field

The invention relates to a needle assembly magazine.

### Background of the Invention

Conventional medicament delivery devices typically require a needle assembly for creating a fluid path between a medicament container and a needle. It is generally recommended to use an unused injection needle for each injection in order to reduce the risk for cross contamination, infection and pain associated with reuse of used needles.

Many users of medicament delivery devices, such as pen injectors and autoinjectors, may be elderly or have reduced dexterity. For these groups, mounting needle assemblies to the delivery device may be problematic if the needle assembly is not oriented corrected, which may disrupt the fluid path for the medicament or may lead to a painful injection process. Further, removing used injection needles can be difficult and may subject the user to the risk of needle stick injuries. Generally, it is recommended that needles be removed with care to avoid needle stick injury.

Thus, there remains a need for an improved needle assembly magazine which facilitates needle assembly mounting and removal on/from a medicament delivery device.

### Summary of the Invention

It is an object of the present invention to provide an improved needle assembly magazine.

In an exemplary embodiment, a needle assembly magazine according to the present invention comprises a body, and a plurality of needle assembly compartments formed in the body. Each needle assembly compartment includes at least one track adapted to receive a follower pin coupled to a needle assembly. The at least one track comprises a substantially horizontal proximal portion coupled to a substantially vertical longitudinal portion coupled to a substantially horizontal distal portion. The needle assembly is restricted to rotational movement relative to the needle assembly compartment when the follower pin is in the proximal portion and the distal portion and restricted to axial movement when the follower pin is in the longitudinal portion.

In an exemplary embodiment, the needle assembly further comprises a spring disposed in each of the needle assembly compartments and applying a biasing force to the needle assembly.

In an exemplary embodiment, the needle assembly further comprises a lid disposed over a first surface of the body, a shield disposed over a second surface of the body, and a stem coupled to the lid and the shield. The stem prevents rotation of the lid relative to the shield but allows rotation of the body relative to the stem. The lid includes an opening adapted to receive a medicament delivery device. The lid includes a window for viewing an indicia disposed on the body adjacent the plurality of needle assembly compartments. The shield includes a radial opening substantially axially aligned with the opening and adapted to receive a needle cover on the needle assembly. A channel adapted to receive a protrusion on the medicament delivery device is formed in a circumference of the opening. The follower pin includes a slot adapted to engage the protrusion.

In an exemplary embodiment, the needle assembly includes at least one removable seal.

In an exemplary embodiment, the at least one track includes at least one backstop allowing movement of the follower pin in the track in a first direction but preventing movement of the follower pin in the track in a second direction opposite the first direction.

In an exemplary embodiment, the at least one track includes a substantially horizontal second proximal portion coupled to a substantially vertical second longitudinal portion coupled to the substantially horizontal distal portion. The needle assembly is restricted to rotational movement in a first rotational direction relative to the needle assembly compartment when the follower pin is in the proximal portion, the distal portion, and the second proximal portion, and restricted to axial movement when the follower pin is in the longitudinal portion and the second longitudinal portion.

In exemplary embodiments, the needle assembly magazine according to the invention reduces the risk of needle stick injuries and facilitates proper and safe mounting and removal of needle assemblies on/from a medicament delivery device. After all of the needle assemblies in the needle assembly magazine have been used, the needle assembly magazine may be discarded.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a schematic top view of an exemplary embodiment of a needle assembly magazine,
- Figure 2: is a schematic longitudinal section of an exemplary embodiment of a needle assembly magazine,
- Figure 3: is a schematic exploded view of an exemplary embodiment of a needle assembly magazine,
- Figure 4: is a schematic perspective detail view of an exemplary embodiment of a body of a needle assembly magazine,
- Figure 5: is a schematic of an exemplary embodiment of a track in a needle assembly compartment of the needle assembly magazine and a follower pin on a needle hub,
- Figure 6: is a schematic perspective detail view of an exemplary embodiment of a needle assembly magazine with an interface for connecting to a medicament delivery device,
- Figure 7: is a schematic perspective view of an exemplary embodiment of a needle assembly magazine and a medicament delivery device in a first position,
- Figure 8: is a schematic perspective view of an exemplary embodiment of a needle assembly magazine with an inserted medicament delivery device in a second position,
- Figure 9: is a schematic perspective view of an exemplary embodiment of a needle assembly magazine with an inserted medicament delivery device in a third locked position,
- Figure 10: is a schematic perspective view of an exemplary embodiment of a needle assembly magazine after removal of a needle cover,
- Figure 11: is a schematic perspective view of a top side of another exemplary embodiment of a needle assembly magazine,
- Figure 12: is a schematic perspective view of an exemplary embodiment of a body of the needle assembly magazine,
- Figure 13: is a schematic longitudinal section an exemplary embodiment of the needle assembly magazine,
- Figure 14: is a schematic of an exemplary embodiment of a track in the needle assembly compartment of the needle assembly magazine and a follower pin on the needle hub, and
- Figure 15: is a schematic of an exemplary embodiment of a track in a needle assembly compartment of yet another exemplary embodiment the needle assembly magazine and a follower pin on a needle hub.

Corresponding parts are marked with the same reference symbols in all figures.

### Detailed Description

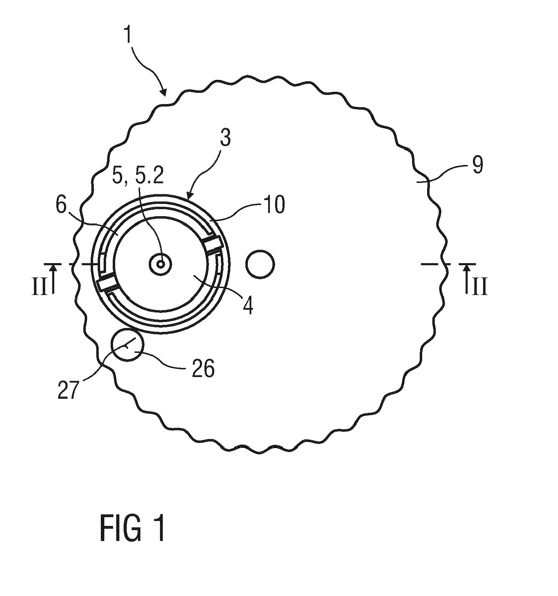

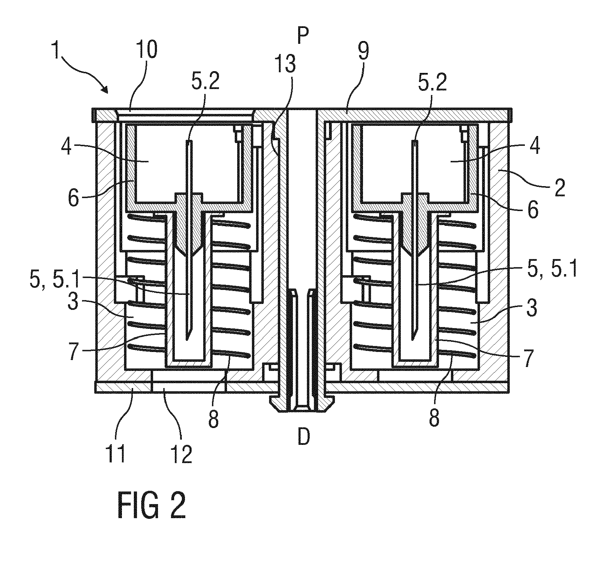

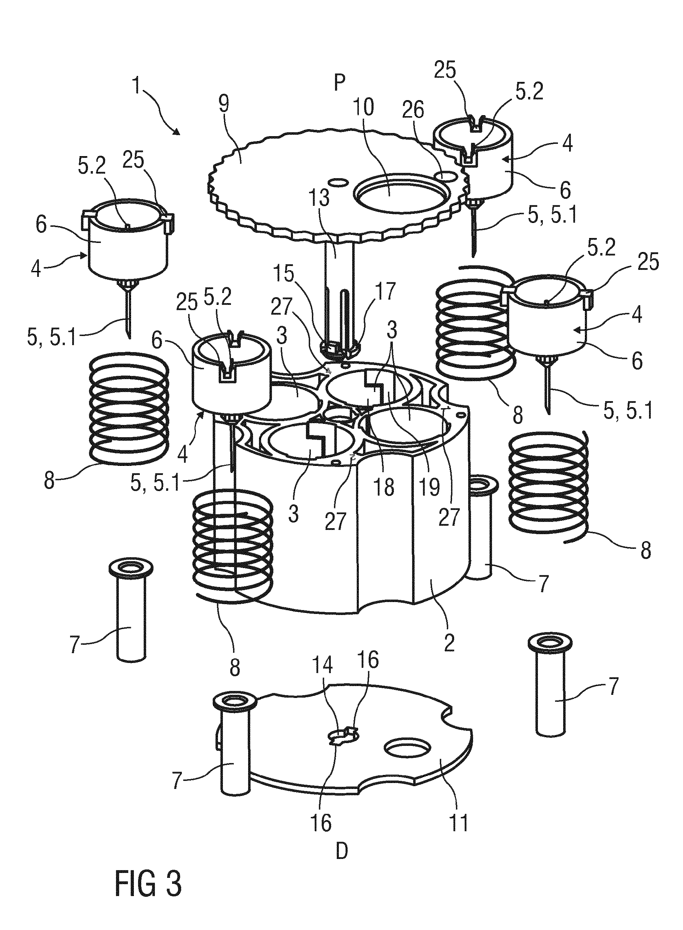

Figure 1 is a schematic top view of an exemplary embodiment of a needle assembly magazine 1. Figure 2 is a corresponding schematic longitudinal section of an exemplary embodiment of a needle assembly magazine. Figure 3 is a corresponding schematic exploded view of an exemplary embodiment of a needle assembly magazine.

Referring to Figure 3, an exemplary embodiment of a needle assembly magazine 1 according to the present invention comprises a body 2 which includes a plurality of needle assembly compartments 3 that are each adapted to receive a needle assembly 4. Each of the needle assembly compartments 3 includes a spring 8 adapted to apply a biasing force on the needle assembly 4 in the needle assembly compartment 3. In the exemplary embodiment, the body 2 includes four needle assembly compartments 3, however those of skill in the art will understand that the body 2 could be modified to receive any number of needle assemblies 4 without departing from the full scope and spirit of the present invention.

In the exemplary embodiment, the needle assembly 4 comprises a needle 5 having a distal portion 5.1 and a proximal portion 5.2, and a hub 6 which supports the needle 5. When the needle assembly 4 is coupled to a medicament delivery device (e.g., a pen injector, an autoinjector, etc.), the distal portion 5.1 is used to pierce an injection site and the proximal portion 5.2 pierces a septum on a medicament container (e.g., a cartridge, an ampoule, a syringe, etc.) to create a fluid path for the medicament. Each needle assembly 4 may further include a needle cover 7 removably arranged over the distal portion 5.1 of the needle 5.

In an exemplary embodiment, a lid 9 is rotatably disposed on the body 2. The lid 9 includes an opening 10 which is sized and shaped to correspond to a size and shape of an opening of each of the needle assembly compartments 3. In use, a distal end of a medicament delivery device may be inserted through the opening 10 to engage/disengage a needle assembly 4. The lid 9 may include one or more grip features which facilitate rotation of the lid 9 relative to the body 2, e.g., for aligning the opening 10 with a given needle assembly compartment 3. The grip features may include a serrated circumference, a handle, a depression, a cover/skin, etc. In an exemplary embodiment, the lid 9 may further include a window 26 which allows visualization of an indicia 27 on the body 2. For example, the indicia 27 may be a number associated with each needle assembly compartment 3 and/or a use indicator (e.g., color, symbol, etc.) to visually indicate that a needle assembly 4 in a given needle assembly compartment 3 has been used or unused.

In an exemplary embodiment, a shield 11 is disposed on the body 2, on an end opposite the lid 9. The shield 11 includes a radial opening 12 which is adapted to allow passage of the needle cover 7. The radial opening 12 has a cross-section substantially equal to a cross-section of the needle cover 7, such that the needle cover 7 can be entirely pulled through the radial opening 12. The shield 11 further includes a central opening 14 which is adapted to receive a stem 13 which is also fixed to the lid 9. The stem 13 may include one or more splines 15 which engage one or more corresponding recesses 16 formed around the central opening 14 to prevent relative rotation of the stem 13 (and the lid 9) and the shield 11. The splines 15 also angularly orient the lid 9 relative to the shield 11 such that the radial opening 12 is aligned with the opening 10. The stem 13 may further include one or more resilient clips 17 which snap-lock to the shield 11 during engagement of the stem 13 and the shield 11.

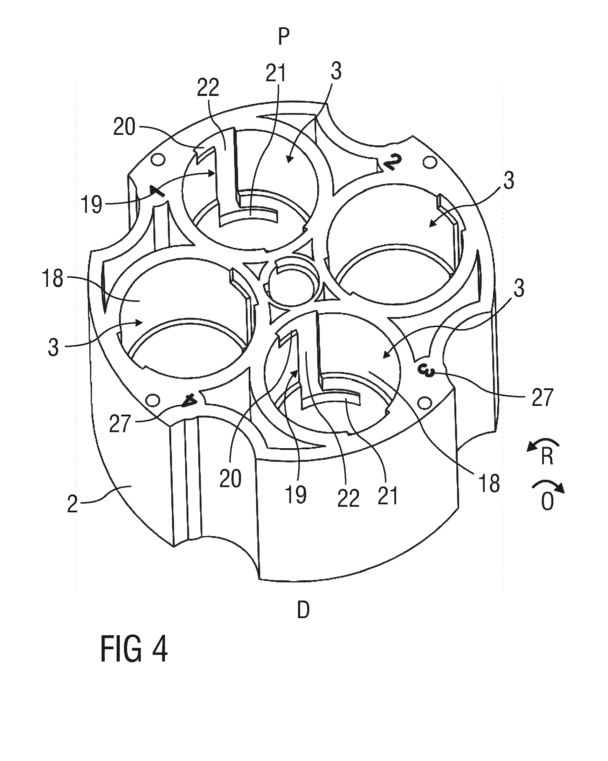

As shown in Figure 4, in an exemplary embodiment, the needle assembly compartment 3 includes at least one track 19 which is adapted to mate with a follower pin 25 on the needle hub 6. In another exemplary embodiment, the follower pin 25 may be formed on a carriage (not shown) which is disposed in needle assembly compartment 3 and adapted to engage a needle assembly 4. The track 19 may comprise a proximal portion 20 coupled to a distal portion 21 by a longitudinal portion 22.

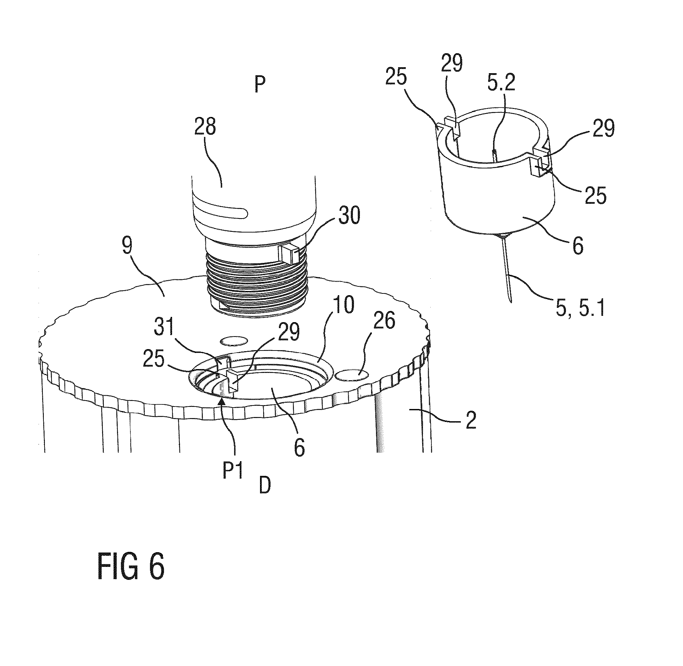

Figure 6 is a schematic perspective detail view of an exemplary embodiment of the needle assembly magazine 1 according to the present invention. A medicament delivery device 28 includes a protrusion 30 adapted to engage the follower pin 25 formed on a needle assembly 4. For example, the follower pin 25 may be formed as a slot 29 which receives the protrusion 30. To ensure that the delivery device 28 is proplery aligned with the needle assembly 4, a channel 31 may be formed in the circumference of the opening 10 on the lid 9. To engage the needle assembly 4, the protrusion 30 on the delivery device 28 should be aligned with the channel 31, which is also aligned with the slot 29 on the follower pin 25.

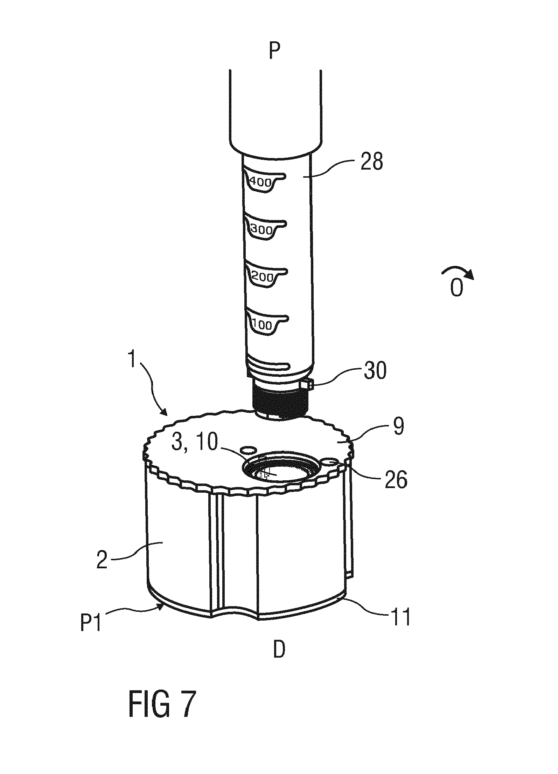

In use, as shown in Figure 7, the delivery device 28 (without a needle assembly 4 engaged thereto) is inserted into a needle assembly compartment 3 via the opening 10. The channel 31 may facilitate alignment of the delivery device 28 with the needle assembly 4 in the needle assembly compartment 3.

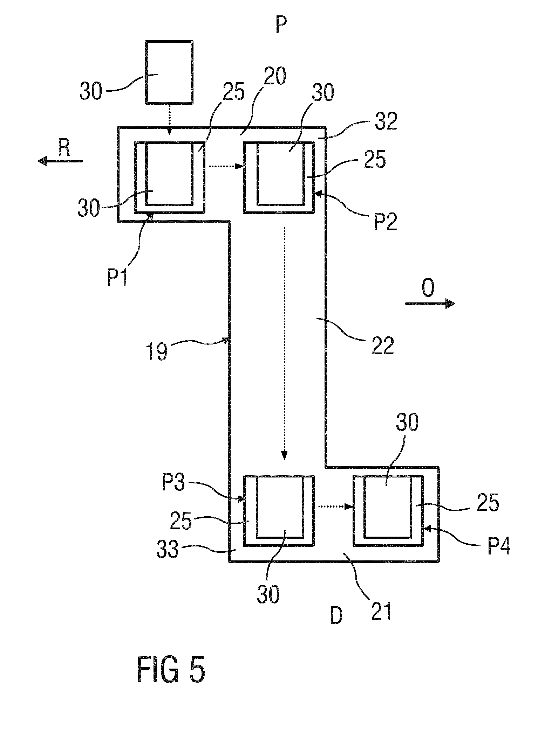

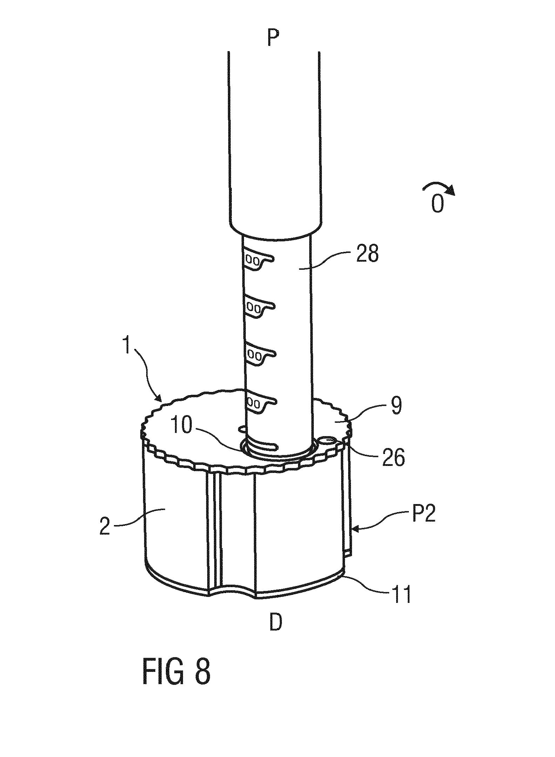

Referring to Figure 5, when the protrusion 30 engages the slot 29 of the follower pin 25, the needle assembly 4 may be in a first position P1 in the proximal portion 20 of the track 19. Because the proximal portion 20 is a horizontal channel, axial force will not displace the needle assembly 4 relative to the needle assembly compartment 3. However, when the delivery device 28 is rotated in a first rotational direction R1, the protrusion 30 rotates the needle assembly 4 into a second position P2 in the proximal portion 20 of the track 19. Then, axial force applied to the delivery device 28 causes translation of the needle assembly 4 relative to the needle assembly compartment 3, in which the follower pin 25 translates within the longitudinal section 22 of the track 19 (shown in Figures 5 and 8) to a third position P3. Translation of the needle assembly 4 from the second position P2 to the third position P3 is against the biasing force of the spring 8.

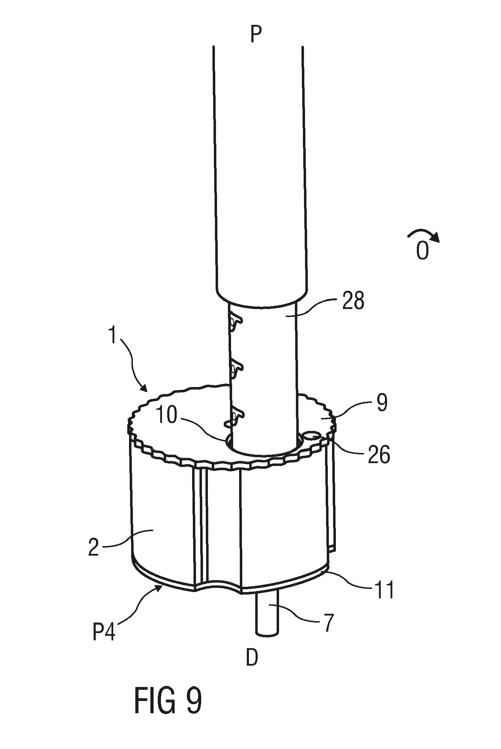

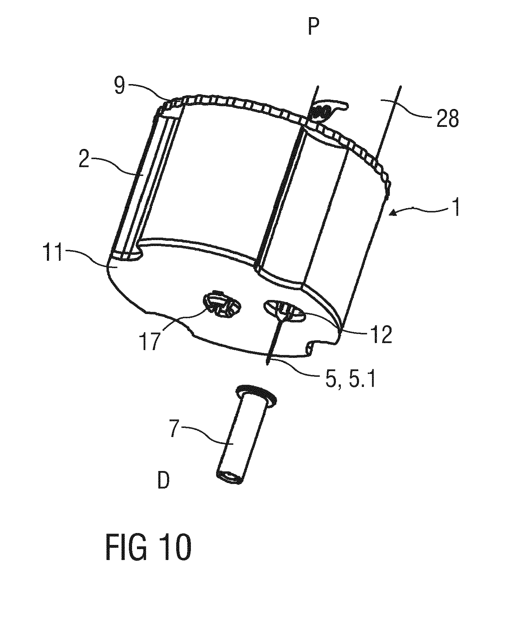

When the needle assembly 4 is in the third position P3, the needle cover 7 extends, at least partially, through the radial opening 12 (as shown in Figure 9). Further rotation of the delivery device 28 causes the needle assembly 4 to move to a fourth position P4 in distal portion 21 of the track 19. Because the distal portion 21 is a horizontal channel, the needle assembly 4, and the delivery device 28 engaged thereto, are maintained in an axial position relative to the needle assembly magazine 1. In this position, the needle cover 7 is substantially wholly exposed through the radial opening 12 and can be removed, exposing the distal portion 5.1 of the needle 5 (as shown in Figure 10). With the distal portion 5.1 of the needle 5 exposed, an injection can be administered.

After injection, the delivery device 28 is rotated in a second rotational direction R2, moving the protrusion 30 and the follower pin 25 along the distal portion 21 of the track 19 from the fourth position P4 to the third position P3. The biasing force in the needle spring 8 pushes the needle assembly 4 (and the delivery device 28 engaged thereto) from the third position P3 to the second position P2 in the track 19. When the needle assembly 4 is in the second position P2, the distal portion 5.1 of the needle 5 is retracted into the body 2. Further rotation of the delivery device 28 in the second rotational direction R2 moves the protrusion 30 and the follower pin 25 from the second position P2 to the first position P1 in the track 19. Because the protrusion 30 is aligned with the channel 31, the delivery device 28 can be removed from the magazine 1.

After the delivery device 28 is removed from the magazine 1, the lid 9 and the shield 11 can be rotated relative to the body 2 to cover the needle assembly compartment 3 containing the used needle assembly 4 and to align the opening 10 and the radial opening 12 with a needle assembly compartment 3 containing an unused needle assembly 4. An unused assembly 4 may be recognized, because the needle assembly 4 may include a seal 34.

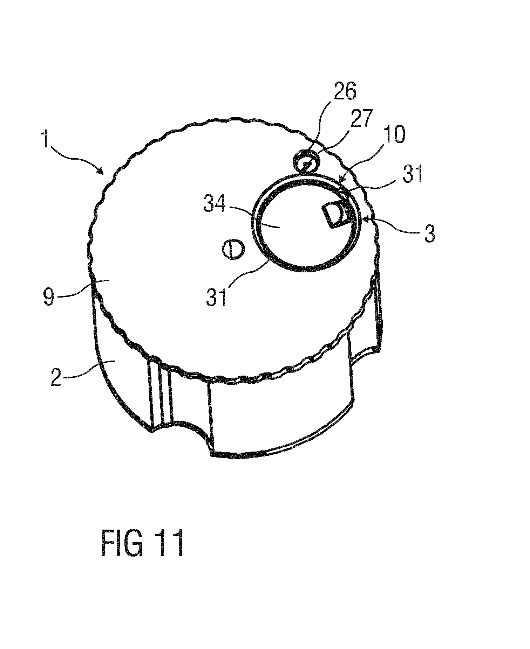

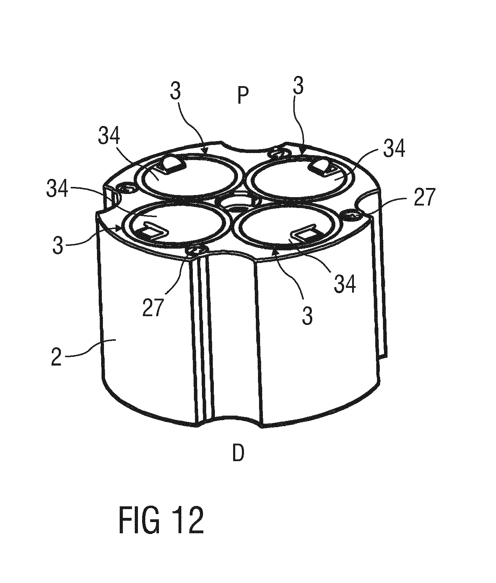

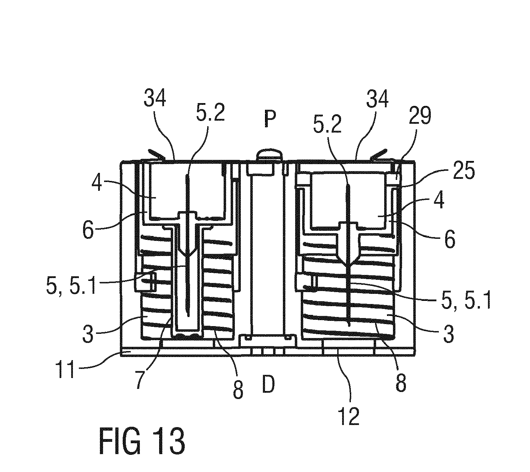

Figure 11 is a schematic perspective view of a top side of another exemplary embodiment of a needle assembly magazine 1 according to the present invention. Figure 12 is a corresponding schematic perspective view of another exemplary embodiment of a body 2 of a needle assembly magazine 1 according to the present invention. Figure 13 is a corresponding schematic longitudinal section of another exemplary embodiment of a needle assembly magazine 1 according to the present invention.

The exemplary embodiment of the needle assembly magazine 1 shown in Figures 11-13 differs from the above-described exemplary embodiments in that seals 34, e.g. from paper or foil, may be attached to the proximal and distal surfaces of each needle assembly compartment 3. A top seal 34 may be arranged over the needle assembly compartment 3 underlying the opening 10 in the lid 9. The top seal 34 may be removed manually before the delivery device 28 is inserted into the needle assembly compartment 3. A bottom seal (not shown) may be arranged over the needle assembly compartment adjacent the radial opening 12 in the shield 11. The needle 5 may then pierce the bottom seal (not illustrated) when the needle 5 is extended from the body 2, e.g., when the needle assembly 4 is moved from the second position P2 to the third position P3 in the track 19. This may ensure sterility of the needle 5. Furthermore, in this exemplary embodiment, a needle cover 7 may not be necessary, saving costs and removing possibility of needle-stick injury.

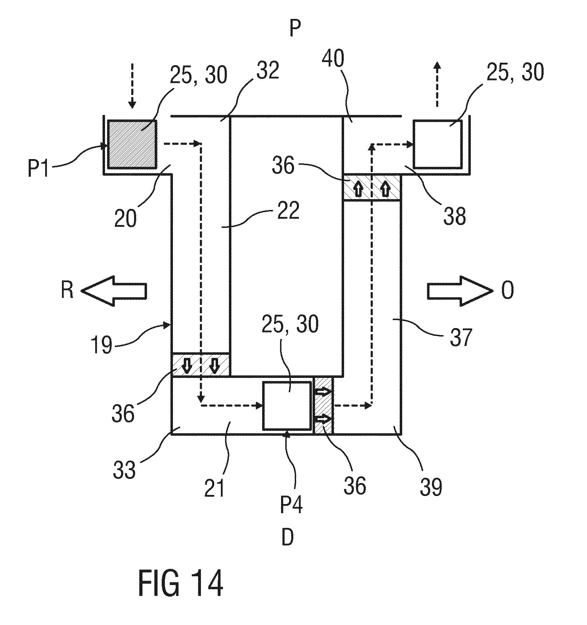

Figure 14 is a schematic of a track 19 in a needle assembly compartment 3 according to another exemplary embodiment of the invention. In this exemplary embodiment, the track 19 in the needle assembly compartment 3 includes one or more backstops 36 which allow the follower pin 25 to move in a first direction in the track 19 but prevent movement in a second direction opposite the first direction.

In another exemplary embodiment, the track 19 may further comprise a return path with a second longitudinal portion 37 and a second proximal portion 38. The backstops 36 may be in each of the longitudinal portions 22 and 32, and the distal portion 21. In this exemplary embodiment, the delivery device 28 may always be rotated in the first rotational direction R1 to engage the needle assembly 4, administer the injection, and disengage the needle assembly 4.

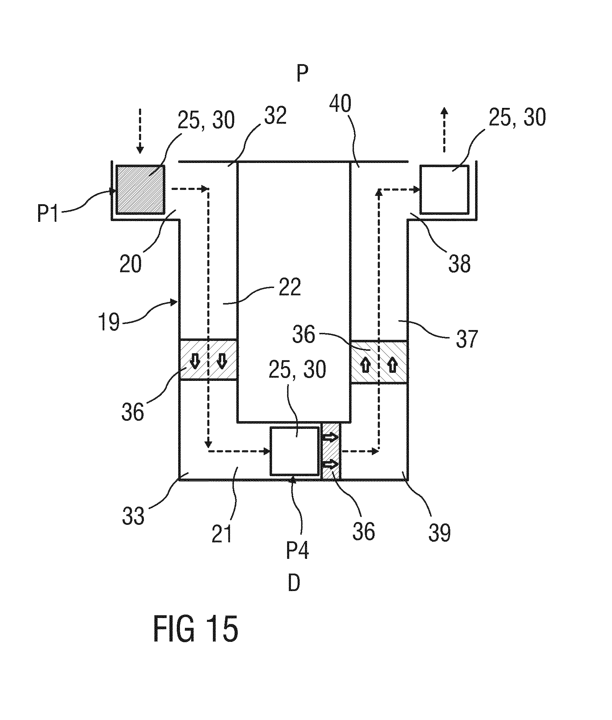

Figure 15 is a schematic of an exemplary embodiment of a track 19 in a needle assembly compartment 3. In contrast to the exemplary embodiment of figure 14, the position of the one way features 36 in the first and second longitudinal portions 22, 37 is varied such that they are encountered by the follower pin 25 immediately prior to, and after, the needle 5 protruding from the needle assembly magazine 1.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle assembly magazine (1) comprising:
a body (2); and
a plurality of needle assembly compartments (3) formed in the body (2), wherein each needle assembly compartment (3) includes at least one track (19) adapted to receive a follower pin (25) coupled to a needle assembly (4),
wherein the at least one track (19) comprises a substantially horizontal proximal portion (20) coupled to a substantially vertical longitudinal portion (22) coupled to a substantially horizontal distal portion (21), and
wherein the needle assembly (4) is restricted to rotational movement relative to the needle assembly compartment (3) when the follower pin (25) is in the proximal portion (20) and the distal portion (21) and restricted to axial movement when the follower pin (25) is in the longitudinal portion (22).

2. The needle assembly magazine (1) according to claim 1 further comprising:
a spring (8) disposed in each of the needle assembly compartments (3) and applying a biasing force to the needle assembly (4).

3. The needle assembly magazine (1) according to any one of the preceding claims further comprising:
a lid (9) disposed over a first surface of the body (2);
a shield (11) disposed over a second surface of the body (2); and
a stem (3) coupled to the lid (9) and the shield (11), wherein the stem (3) prevents rotation of the lid (9) relative to the shield (11) but allows rotation of the body (2) relative to the stem (3).

4. The needle assembly magazine (1) according to claim 3 wherein the lid (9) includes an opening (10) adapted to receive a medicament delivery device (28).

5. The needle assembly magazine (1) according to claim 3 wherein the lid (9) includes a window (26) for viewing an indicia (27) disposed on the body (2) adjacent the plurality of needle assembly compartments (3).

6. The needle assembly magazine (1) according to claims 3 and 4 wherein the shield (11) includes a radial opening (12) substantially axially aligned with the opening (10) and adapted to receive a needle cover (7) on the needle assembly (4).

7. The needle assembly magazine (1) according to claim 4 wherein a channel (31) adapted to receive a protrusion (30) on the medicament delivery device (28) is formed in a circumference of the opening (10).

8. The needle assembly magazine (1) according to claim 7, wherein the follower pin (25) includes a slot (29) adapted to engage the protrusion (30).

9. The needle assembly magazine (1) according to any one of the preceding claims, wherein the needle assembly (4) includes at least one removable seal (34).

10. The needle assembly magazine (1) according to any one of the preceding claims, wherein the at least one track (19) includes at least one backstop (36) allowing movement of the follower pin (25) in the track (19) in a first direction but preventing movement of the follower pin (25) in the track (19) in a second direction opposite the first direction.

11. The needle assembly magazine (1) according to any one of the preceding claims, wherein the at least one track (19) includes a substantially horizontal second proximal portion (38) coupled to a substantially vertical second longitudinal portion (37) coupled to the substantially horizontal distal portion (21), and
wherein the needle assembly (4) is restricted to rotational movement in a first rotational direction (R1) relative to the needle assembly compartment (3) when the follower pin (25) is in the proximal portion (20), the distal portion (21), and the second proximal portion (38), and restricted to axial movement when the follower pin (25) is in the longitudinal portion (22) and the second longitudinal portion (37).
